# EUROPEAN PATENT SPECIFICATION

(11) **EP 3 650 052 B1**
(45) Date of publication and mention of the grant of the patent: **22.10.2025**
(21) Application number: 18827959.0
(22) Date of filing: 26.06.2018
(51) Int. Cl.: A61L 15/22, A61F 13/00

(54) **PATCH FOR REGENERATING BIOLOGICAL TISSUES AND METHOD FOR PRODUCING SAME**
PFLASTER ZUR REGENERIERUNG VON BIOLOGISCHEM GEWEBE UND VERFAHREN ZU SEINER HERSTELLUNG
PATCH POUR LA RÉGÉNÉRATION DE TISSUS BIOLOGIQUES ET PROCÉDÉ DE FABRICATION DE CELUI-CI

(30) Priority: 03.07.2017 ES 201730877
(43) Date of publication of application: 13.05.2020
(73) Proprietor: Viscofan, S.A., 31192 Tajonar (Navarra) (ES)
(72) Inventor: HUSS, Annika, 67227 Frankenthal (DE); BUSCH, Silke, 69115 Heidelberg (DE); QUINTANA FRIGOLA, Lluís, 64646 Heppenheim (DE); PRÓSPER CARDOSO, Felipe, 31190 Cizur Menor (Navarra) (ES); GAVIRA GÓMEZ, Juanjo, 31192 Mutilva (Navarra) (ES); RÁBAGO JUAN-ARACIL, Gregorio, 31008 Pamplona (Navarra) (ES)
(74) Representative: Herrero & Asociados, S.L.
(86) International application number: PCT/ES2018/070451
(87) International publication number: WO 2019/008203

(56) References cited:
- WO-A1-2011/003422
- WO-A1-2011/003422
- WO-A1-2013/135544
- WO-A1-2015/050749
- WO-A1-93/10731
- WO-A2-2014/070903
- ES-T3- 2 618 404
- US-A- 3 376 869
- US-A1- 2003 139 697
- US-A1- 2013 142 860
- US-B1- 7 396 537

## Description

### TECHNICAL FIELD OF THE INVENTION

The present invention relates to a patch for regenerating biological tissues in patients and to the method of manufacturing the same; this patch belongs to the surgical and hospital sector.

### BACKGROUND OF THE INVENTION

As an introduction, it is known that the use and application of patches for regenerating biological tissue in patients is being increasingly used wherein said patches are particularly a type of collagen sheet, currently called CCC; whereon controlled cell growth is effected such that a regenerative character of the patch itself is achieved. This extended use and application are due to the fact that in many surgical interventions, incisions and injuries are made on the organs of the patients with the aim of reaching the target area of the surgery. Said injuries involve a need for cicatrisation of said operated organs, which involves a long stay in a hospital room, in addition to the physical discomfort suffered by the patient. Furthermore, said injuries require complex cicatrisation, either due to the difficulty of the intervention or due to the very physiognomy of the patient, since they can have a lower number of cicatrising biological agents depending on the physical condition; this can involve a certain risk to the health of the patient.

Therefore, in order to reduce the cicatrisation time of the injuries caused and to reduce the risk of possible health problems in the patient, the use and application technique of CCC collagen patches is known for generating organ tissues comprising, as the name suggests, collagen protein sheets in charge of transporting material for the cells and converting into a substituted body membrane which degrades after a few weeks. Owing to this, the collagen is responsible for accelerating the regeneration and cicatrisation process of said injuries in the patient, thereby reducing the hospital stay and the risk of internal tearing due to the poor cicatrisation of the affected organs.

Even though it is very extensively used and the aim thereof of regeneration is appropriate for the previously mentioned advantages, it presents a significant disadvantage linked to how to apply it to the organs of the patients. This is due to the fact that the CCC collagen patches used currently have a deformable flexible tissue morphology which can be adapted to any type of surface according to the injury of the biological organ that is to be regenerated. However, this flexibility and adaptability is counterproductive when the surgeon responsible should introduce it and place it on said organ; surgeons usually handle sterilised surgical clamps that secure and grip, using one or several ends, the collagen patch and transport this patch to the affected organ such that the patch is folded and should be unfolded for suitable application which involves a delay in the surgery time and can also involve biological contamination of the patch since it must be unfolded and handled.

The patent publication US7396537 discloses a cell delivery patch adapted for implantation in the heart of an animal, comprising: a collagen hydrogel layer having tissue cells for delivery and biologically active molecules disposed of therein and/or thereon that enable said tissue cells to grow and/or differentiate; an intermediate scaffolding layer that comprises a biodegradable porous mesh that forms a composite construct with said collagen hydrogel layer; and a reinforcement layer adjacent to said intermediate layer, said reinforcement layer comprising a material extending substantially over a cross-sectional dimension. The patch is reinforced with a layer of non-biodegradable support material.

The patent application WO2011003422 relates to a biodegradable surgical implant far support, augmentation and regeneration of living tissue in a subject, comprising: a) a synthetic biodegradable homogenous sheet of scaffold which can be made of collagen, b) one or more biodegradable reinforcing members made of biodegradable fibres and/or threads in a pattern selected from the group consisting of: triangles, circles, connecting waves, non-connecting waves, and overlapping waves. The document does not describe the use of a reinforcement layer made of collagen.

The patent application WO2014070903 discloses a biocompatible tissue graft comprising: i) a first layer of a bioremodelable collageneous material; ii) a second layer of biocompatible synthetic or natural remodelable or substantially remodelable material attached to the first layer; and iii) at least one fiber stitched in a reinforcing pattern in the first layer and/or second layer to mitigate tearing of the graft, improve fixation retention of the graft, and/or limit cyclic stretching of the graft. This patent application does not relate to a collagen reinforcement layer.

The patent application WO9310731 discloses the use of cell patches based on a membrane made of collagen. The reinforcement of the cell patches described is not made of the same material as the membrane.

Therefore, given the disadvantages associated with the difficulties in applying patches for regenerating biological tissues in patients, it is necessary to find a new patch that can be applied on any type of surface of one or several biological organs that has a high adaptability and biological tissue-regeneration properties and which also facilitates and improves the application and placement techniques of said patch on the damaged organ and reduces the application time of said patch.

### DESCRIPTION OF THE INVENTION

The present invention relates to a patch for regenerating biological tissues in patients, comprising:
- a membrane made of collagen comprising cells with a regenerative character; and
- also comprises a reinforcement element configured to increase the rigidity of said membrane, preventing the deformation of said membrane during the handling thereof wherein said at least one reinforcement element is manufactured with the same material as said membrane made of collagen.

Wherein, for clarification purposes, it is observed that the membrane is made of a collagen material and which has a plurality of cells with a regenerative character for biological tissue since the method of regenerating said cells is carried out by growing said cells in the collagen membrane.

Therefore, thanks to the existence of said at least one reinforcement element, all the problems related to the step of applying the patch object of the invention on the organ of the patient are completely resolved; since this reinforcement element prevents the collagen membrane folding on itself, achieving a reduction in the application time of the patch from when it is taken from the sterilised container thereof to when it is applied to the organ of the patient.

In this respect, and in relation to said reinforcement element, different embodiment options are achieved, configured to increase, in any case, the rigidity of the collagen membrane connected to the patch object of the invention wherein:
- said at least one reinforcement element comprises a perimeter frame with respect to said membrane such that it prevents the bending of the patch since the preferred securing by the surgeon with respect to the patch is carried out along the perimeter contour of the same and, given that it is reinforced. it makes the easy and effective folding over on itself more difficult.
- said at least one reinforcement element comprises a crossed attachment between the ends of said collagen membrane; once again increasing the rigidity of the entire patch in a manner similar to a triangular bar structure which results in the non-deformability of the patch and can be complemented by the described perimeter frame which ensures the advantage of the application time mentioned above.

Furthermore, with the aim of simplifying the design and manufacture of the patch object of the invention, is considered the one wherein said at least one reinforcement element is manufactured with the same material as said collagen membrane. This also ensures that there are no incompatibilities between the materials forming said patch which is a homogenous material and increases the guarantee of acceptance and reabsorption of the patch with respect to the biological organ of the patient.

Moreover, the method of manufacturing the patch object of the invention is described, comprising the following steps:
a) manufacturing a collagen membrane;
b) manufacturing said reinforcement element;
c) placing said at least one reinforcement element with respect to said membrane;
d) sterilising the membrane; and
e) adding cells with a regenerative character on said membrane.

Wherein the aforementioned steps involve an easy and logical method of manufacture which does not involve a substantial increase to the cost compared to the existing prior art; which can have different possibilities of manufacture with respect to how to design said at least one reinforcement element.

It should be pointed out that, since said patch falls within the surgical sector, the preferred option is the one wherein the method of manufacturing the patch comprises, subsequent to step c) and prior to step e), sterilising the assembly formed by the collagen membrane and said at least one reinforcement element; ensuring hygienic and healthy use in the patient affected.

With respect to the reinforcement element, it can be made of an element external to the collagen membrane and it is described how in step c), the placement of said at least one reinforcement element with respect to said membrane is carried out by means of adhering two elements with water, for example. Or an option may be considered wherein and during step c) the placement of said at least one reinforcement element with respect to said membrane is carried out in two sub steps:
c1) wetting the membrane, placing the reinforcement; and
c2) irradiating the membrane and the reinforcement element by means of gamma radiation.

Furthermore, in contrast to the use of a reinforcement element external to the membrane; the preferred option is described wherein the steps a) and b) of manufacturing the membrane and said at least one reinforcement element are carried out in a single step since said at least one reinforcement element is part of the membrane itself, and both entities being collagen; and wherein the step c) of placing said at least one reinforcement element with respect to said membrane is carried out by bending at least one of the borders of said membrane. This simplifies the manufacture thereof and ensures the homogeneity of the collagen patch material thus obtained.

In a similar manner, and which can be considered complementary or independent embodiments, the option is described wherein the steps a) and b) of manufacturing the membrane and said at least one reinforcement element are also carried out in a single step, since said at least one reinforcement element is part of the collagen membrane itself, but wherein said step c) of placing said at least one reinforcement element with respect to said membrane is carried out by bending at least the internal surface of said membrane.

Therefore, with the proposed invention, a patch is obtained for regenerating biological tissues in patients which can be applied to any type of biological organ with maximum guarantees of success, reducing the application time; and it also highlights the easy manufacture of the same and the wide variety of geometric shapes to be implemented according to the location at which said patch object of the invention will be applied.

### DESCRIPTION OF THE DRAWINGS

In order to complement the description being given and with the aim of aiding a better understanding of the features of the invention, according to a preferred exemplary embodiment of the same, as an integral part of said description, a drawing is included wherein the following has been represented in an illustrative and non-limiting manner:

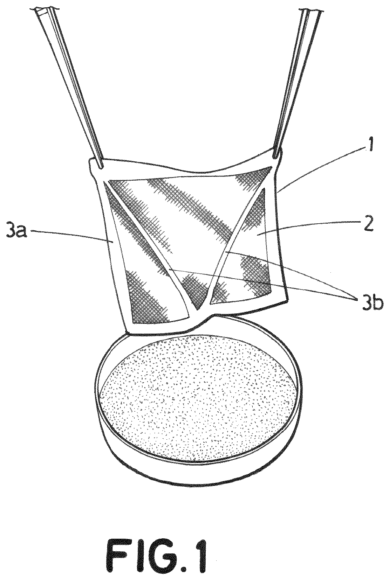

Figure 1 shows a three-dimensional view of the patch for regenerating biological tissues in patients object of the invention, being ensured by surgical material.

### PREFERRED EMBODIMENT OF THE INVENTION

Figure 1 shows a patch (1) for regenerating biological tissues in patients object of the invention comprising:
- a membrane (2) made of collagen with a plurality of cells with a regenerative character; and
- two reinforcement elements (3a, 3b), each one configured to increase the rigidity of said membrane (2), preventing the deformation of said membrane (2) during the handling thereof.

Said reinforcement elements (3a, 3b) are defined by a perimeter frame (3a) with respect to said membrane (2); and by a pair of crossed attachments (3b) between the ends of said membrane (2); which increase the structural rigidity of the entire patch (1) in a highly effective manner since said reinforcement elements (3a, 3b) are manufactured with the same collagen material as said membrane (2), simplifying to a large extent the installation process thereof during surgery.

In this respect, the preferred methods of manufacture of the patch (1) for regenerating biological tissues in patients, comprise the following steps:
a) manufacturing the collagen membrane (2) and the reinforcement elements (3a, 3b);
b) placing and generating said reinforcement elements (3a, 3b) with respect to said membrane (2). In which the step b) of placing said reinforcement elements (3a, 3b) with respect to said membrane (2) is carried out by bending each one of the borders of said membrane (2) in order to implement the perimeter frame (3a); and each one of the crossed attachments (3b) is carried out by folding a portion of the internal surface of said membrane (2);
c) sterilising the assembly formed by the membrane (2) and the reinforcement elements (3a, 3b); and lastly
d) adding cells with a regenerative character on said membrane (2).

In light of the present description and figure, a person with skill in the art could understand that the embodiments of the invention described can be combined in multiple manners within the object of the invention. The invention has been described according to some preferred embodiments of the same, but for the person with skill in the art it will be evident that there can be multiple variations made in said preferred embodiments without departing from the object of the claimed invention.

## Claims

1. A patch (1) for regenerating biological tissues in patients **characterised by** comprising membrane (2) made of collagen which comprises cells with a regenerative character and also comprises a reinforcement element (3a, 3b) configured to increase the rigidity of said membrane (2), preventing the deformation of said membrane (2) during the handling thereof wherein said at least one reinforcement element (3a, 3b) is manufactured with the same material as said membrane made of collagen.

2. The patch (1) according to claim 1, **characterised in that** said at least one reinforcement element (3a, 3b) comprises a perimeter frame (3a) belonging to said membrane (2).

3. The patch (1) according to claim 2, **characterised in that** comprises a reinforcement configured in "V" (3b) attachment between ends of said membrane (2).

4. A method of manufacturing a patch (1) for regenerating biological tissues in patients as defined in claim 1, **characterised in that** it comprises the following steps:
a) manufacturing a collagen membrane (2);
b) manufacturing at least one reinforcement element (3a, 3b);
c) placing said at least one reinforcement element (3a, 3b) with respect to said membrane (2);
d) sterilising the membrane; and
e) adding cells with a regenerative character on said membrane (2).

5. The method of manufacturing a patch (1) according to claim 4, **characterised in that** subsequent to step c) and prior to step e), the assembly formed by the collagen membrane (2) and said at least one reinforcement element (3a, 3b) are sterilised.

6. The method of manufacturing a patch (1) according to any of claims 4 and 5, **characterised in that** in step c), placing said at least one reinforcement element (3a, 3b) with respect to said membrane (2) is carried out in two sub steps:
c1) wetting the membrane (2); and
c2) irradiating the membrane (2) and the reinforcement element (3a, 3b) by means of gamma radiation.

7. The method of manufacturing a patch (1) according to any of claims 4 and 5, **characterised in that** the steps a) and b) of manufacturing the membrane (2) and said at least one reinforcement element (3a, 3b) are carried out in one single step since said at least one reinforcement element (3a, 3b) is part of the membrane (2) itself and both entities being collagen; and wherein the step c) of placing said at least one reinforcement element (3a, 3b) with respect to said membrane (2) is carried out bending at least one of the borders of said membrane (2).

8. The method of manufacturing a patch (1) according to any of claims 4 to 7, **characterised in that** steps a) and b) of manufacturing the membrane (2) and said at least one reinforcement element (3a, 3b) are carried out in one single step since said at least one reinforcement element (3a, 3b) is part of the membrane (2) itself and both entities being collagen; and wherein the step c) of placing said at least one reinforcement element (3a, 3b) with respect to said membrane (2) is carried out by folding at least a portion of the internal surface of said membrane (2).

## Patentansprüche

1. Pflaster (1) zur Regenerierung von biologischen Geweben bei Patienten, **dadurch gekennzeichnet, dass** es Membran (2), die aus Kollagen gemacht ist und Zellen mit einem regenerativen Charakter umfasst, und auch ein Verstärkungselement (3a, 3b) umfasst, das ausgestaltet ist, um die Steifheit der Membran (2) zu erhöhen, wodurch die Verformung der Membran (2) während ihrer Handhabung verhindert wird, wobei das mindestens eine Verstärkungselement (3a, 3b) mit dem gleichen Material wie die aus Kollagen gemachte Membran gefertigt wird.

2. Pflaster (1) nach Anspruch 1, **dadurch gekennzeichnet, dass** das mindestens eine Verstärkungselement (3a, 3b) einen Umfangsrahmen (3a) umfasst, der zu der Membran (2) gehört.

3. Pflaster (1) nach Anspruch 2, **dadurch gekennzeichnet, dass** es eine als "V"-Befestigung (3b) ausgestaltete Verstärkung zwischen Enden der Membran (2) umfasst.

4. Verfahren zur Fertigung eines Pflasters (1) zum Regenerieren von biologischen Geweben bei Patienten, wie in Anspruch 1 definiert, **dadurch gekennzeichnet, dass** es die folgenden Schritte umfasst:
a) Fertigen einer Kollagenmembran (2);
b) Fertigen mindestens eines Verstärkungselements (3a, 3b);
c) Platzieren des mindestens einen Verstärkungselements (3a, 3b) in Bezug zu der Membran (2);
d) Sterilisieren der Membran; und
e) Zufügen von Zellen mit einem regenerativen Charakter auf der Membran (2).

5. Verfahren zur Fertigung eines Pflasters (1) nach Anspruch 4, **dadurch gekennzeichnet, dass** nachfolgend zu Schritt c) und vor Schritt e) die durch die Kollagenmembran (2) und das mindestens eine Verstärkungselement (3a, 3b) gebildete Anordnung sterilisiert wird.

6. Verfahren zur Fertigung eines Pflasters (1) nach einem der Ansprüche 4 und 5, **dadurch gekennzeichnet, dass** in Schritt c) Platzieren des mindestens einen Verstärkungselements (3a, 3b) in Bezug zu der Membran (2) in zwei Teilschritten durchgeführt wird:
c1) Benetzen der Membran (2); und
c2) Bestrahlen der Membran (2) und des Verstärkungselements (3a, 3b) mittels gamma-Strahlung.

7. Verfahren zur Fertigung eines Pflasters (1) nach einem der Ansprüche 4 und 5, **dadurch gekennzeichnet, dass** die Schritte a) und b) des Fertigens der Membran (2) und des mindestens einen Verstärkungselements (3a, 3b) in einem Einzelschritt durchgeführt werden, da das mindestens eine Verstärkungselement (3a, 3b) Teil der Membran (2) selbst ist und beide Komponenten Kollagen sind; und wobei der Schritt c) des Platzierens des mindestens einen Verstärkungselements (3a, 3b) in Bezug zu der Membran (2) durchgeführt wird, indem mindestens eine der Grenzen der Membran (2) gebogen wird.

8. Verfahren zur Fertigung eines Pflasters (1) nach einem der Ansprüche 4 bis 7, **dadurch gekennzeichnet, dass** Schritte a) und b) des Fertigens der Membran (2) und des mindestens einen Verstärkungselements (3a, 3b) in einem Einzelschritt durchgeführt werden, da das mindestens eine Verstärkungselement (3a, 3b) Teil der Membran (2) selbst ist und beide Komponenten Kollagen sind; und wobei der Schritt c) des Platzierens des mindestens einen Verstärkungselements (3a, 3b) in Bezug zu der Membran (2) durchgeführt wird, indem mindestens ein Abschnitt der Innenfläche der Membran (2) gefaltet wird.

## Revendications

1. Patch (1) pour la régénération de tissus biologiques chez des patients **caractérisé en ce qu'**il comprend une membrane (2) en collagène qui comprend des cellules à caractère régénérateur et comprend également un élément de renfort (3a, 3b) configuré pour augmenter la rigidité de ladite membrane (2), empêchant la déformation de ladite membrane (2) lors de sa manipulation, dans lequel ledit au moins un élément de renfort (3a, 3b) est fabriqué avec le même matériau que ladite membrane en collagène.

2. Patch (1) selon la revendication 1, **caractérisé en ce que** ledit au moins un élément de renfort (3a, 3b) comprend un cadre périphérique (3a) appartenant à ladite membrane (2).

3. Patch (1) selon la revendication 2, **caractérisé en ce qu'**il comprend un renfort configuré en une fixation en « V » (3b) entre des extrémités de ladite membrane (2).

4. Procédé de fabrication d'un patch (1) pour la régénération de tissus biologiques chez des patients selon la revendication 1, **caractérisé en ce qu'**il comprend les étapes suivantes :
a) la fabrication d'une membrane de collagène (2) ;
b) la fabrication d'au moins un élément de renfort (3a, 3b) ;
c) le positionnement dudit au moins un élément de renfort (3a, 3b) par rapport à ladite membrane (2) ;
d) la stérilisation de la membrane ; et
e) l'ajout de cellules à caractère régénérateur sur ladite membrane (2).

5. Procédé de fabrication d'un patch (1) selon la revendication 4, **caractérisé en ce qu'**après l'étape c) et avant l'étape e), l'ensemble formé par la membrane de collagène (2) et ledit au moins un élément de renfort (3a, 3b) est stérilisé.

6. Procédé de fabrication d'un patch (1) selon l'une quelconque des revendications 4 et 5, **caractérisé en ce qu'**à l'étape c), le positionnement dudit au moins un élément de renfort (3a, 3b) par rapport à ladite membrane (2) est réalisé en deux sous-étapes :
c1) le mouillage de la membrane (2) ; et
c2) l'irradiation de la membrane (2) et de l'élément de renfort (3a, 3b) au moyen de rayons gamma.

7. Procédé de fabrication d'un patch (1) selon l'une quelconque des revendications 4 et 5, **caractérisé en ce que** les étapes a) et b) de fabrication de la membrane (2) et dudit au moins un élément de renfort (3a, 3b) sont réalisées en une seule étape puisque ledit au moins un élément de renfort (3a, 3b) fait partie de la membrane (2) elle-même et les deux entités sont du collagène ; et dans lequel l'étape c) de positionnement dudit au moins un élément de renfort (3a, 3b) par rapport à ladite membrane (2) est réalisée par pliage d'au moins l'une des bordures de ladite membrane (2).

8. Procédé de fabrication d'un patch (1) selon l'une quelconque des revendications 4 à 7, **caractérisé en ce que** les étapes a) et b) de fabrication de la membrane (2) et dudit au moins un élément de renfort (3a, 3b) sont réalisées en une seule étape puisque ledit au moins un élément de renfort (3a, 3b) fait partie de la membrane (2) elle-même et les deux entités sont du collagène ; et dans lequel l'étape c) de positionnement dudit au moins un élément de renfort (3a, 3b) par rapport à ladite membrane (2) est réalisée par pliage d'au moins une partie de la surface interne de ladite membrane (2).
